(19) **Europäisches Patentamt European Patent Office Office européen des brevets**

(11) **EP 4 311 529 B1**

(12) **EUROPEAN PATENT SPECIFICATION**

(45) Date of publication and mention of the grant of the patent:
**28.08.2024 Bulletin 2024/35**

(21) Application number: **22186999.3**

(22) Date of filing: **26.07.2022**

(51) International Patent Classification (IPC):
**A61F 13/42** *(2006.01)* **G01N 27/12** *(2006.01)*

(52) Cooperative Patent Classification (CPC):
**A61F 13/42;** A61F 2013/424; G01N 27/121;
G01N 27/227

(54) **SMART ABSORBENT ARTICLES WITH AUTOMATED STOOL AND URINE DETECTION**

INTELLIGENTE ABSORBIERENDE ARTIKEL MIT AUTOMATISCHER STUHL- UND URINERKENNUNG

ARTICLES ABSORBANTS INTELLIGENTS AVEC DÉTECTION AUTOMATISÉE DE SELLES ET D'URINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**31.01.2024 Bulletin 2024/05**

(73) Proprietors:
• **Ontex BV**
  **9255 Buggenhout (BE)**
• **Ontex Group NV**
  **9320 Erembodegem (BE)**

(72) Inventor: **Hellmold, Jens**
  **59269 Beckum (DE)**

(74) Representative: **Macchetta, Andrea**
  **Ontex BV**
  **Korte Keppestraat 21**
  **9320 Erembodegem-Aalst (BE)**

(56) References cited:
  **EP-A1- 3 415 130    WO-A2-2018/228822**

EP 4 311 529 B1

**Description**

**TECHNICAL FIELD**

The present disclosure is directed to an absorbent article for personal hygiene (such as baby or adult diapers, pants or incontinence briefs or pads) and a system for monitoring thereof.

**BACKGROUND**

Absorbent articles for personal hygiene are designed to absorb and contain bodily exudates, such as a large quantity of urine. Non-limiting examples of disposable absorbent articles include diapers, pants, training pants, pADL, adult incontinence products, and feminine hygiene products (including, for example, sanitary napkins and tampons). Other examples of disposable absorbent articles include bandages and wound dressings. In some embodiments, for example, an absorbent article comprises several layers providing different functions, for example a topsheet, a backsheet and in-between an absorbent core, among other layers.

The function of the absorbent core is to absorb and retain the exudates for a prolonged amount of time, for example overnight for a diaper, minimize re-wet to keep the wearer dry and avoid soiling of clothes or bed sheets. The majority of currently marketed absorbent articles comprise as absorbent material a blend of comminuted wood pulp with superabsorbent polymers (SAP) in particulate form, also called absorbent gelling materials (AGM), see for example U.S. Pat. No. 5,151,092 (Buell). Absorbent articles having a core consisting essentially of SAP as absorbent material (so called "airfelt-free" cores) have also been proposed but are less common than traditional mixed cores (see e.g. WO2008/155699 and WO2012/052172).

A number of disclosures exist (see for example EP2496197B1, EP2739254B1, and EP2582341B1) directed to sensors to sense a condition such as temperature from body or moisture from incontinence. The sensor comprises a signal processing unit, a transmitter and a power supply, typically in form of a battery. These elements are arranged on a flexible substrate in low profile enabling disposition adjacent to the human body. A complex series of mathematical and statistical manipulations are then needed in order to determine wetness events and wetness levels.

While such devices allow monitoring conditions of the human body and can also be used as a moisture sensor, it represents also relatively costly solution. It would not be seen appropriate to dispose of the sensor together with a (disposable) absorbent article. If the sensor, however, is to be reused, the sensing area has potentially been exposed to moisture. Therefore this concept does not allow for simple usage.

Examples of articles that provide improvements to the above drawbacks are disclosed in EP3415130, WO/2018/228822, WO/2018/229017, EP3461257, and EP3451988.

A need nevertheless exists for improved monitoring of stool in a cost effective manner and with limited risk of false positives, and yet allow for accurate urine detection to not be compromised.

**SUMMARY**

In a first aspect the disclosure relates to an absorbent article for personal hygiene such as a diaper, or training pant, or incontinence insert, the absorbent article comprising: a topsheet; a backsheet, wherein the topsheet is liquid permeable and the backsheet is substantially liquid impermeable; an absorbent core positioned between said topsheet and backsheet; a first indicator for indicating the presence of exudates and being positioned on a body-facing side of said backsheet, said first indicator comprising an electrically conductive material, wherein said article comprises a second indicator for indicating the presence of exudates and being positioned on a garment-facing side of said backsheet such that the first and second indicators are separated from each other at least in a thickness direction, said second indicator comprising an electrically conductive material, and wherein said first indicator comprises a first application pattern and said second indicator comprises a second application pattern, wherein said first and second application patterns are different.

In a second aspect the disclosure relates to a kit comprising a plurality of absorbent articles herein described and a detection device operable with any one of said absorbent articles.

**BRIEF DESCRIPTION OF THE FIGURES**

FIG. 1 is a schematic top view of an absorbent article according to an embodiment of the present disclosure in the form of a diaper.

FIG. 2 is a schematic bottom view of an absorbent article according to an embodiment of the present disclosure in the form of a diaper.

FIG. 3 is a schematic top view of an absorbent article according to an embodiment of the present disclosure in the form of a diaper but showing a second indicator positioned on the garment facing side of the diaper being viewable.

FIG. 4 is a schematic cross-sectional view of an exemplary capacitor.

FIG. 5 is a schematic planar view of a second indicator according to an embodiment herein.

**DETAILED DESCRIPTION**

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.

The expression "% by weight" (weight percent), here and throughout the description unless otherwise defined, refers to the relative weight of the respective component based on the overall weight of the formulation.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's garments or undergarments when the absorbent article is worn.

As used herein, the term "absorbent article" refers to disposable devices such as infant or adult diapers or pads, pants, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

The absorbent articles of the disclosure will be further illustrated in the below description and in the Figures in the form of a taped diaper, though all embodiments described herein may equally be applied onto absorbent articles in the form of pants (or even in the form of feminine hygiene or incontinence liners). Nothing in this description should be however considered limiting the scope of the claims unless explicitly indicated otherwise. Unless indicated otherwise, the description refers to the dry article, i.e. before use and conditioned at least 24 hours at 21° C.+/-2° C. and 50+/-20% Relative Humidity (RH).

A "nonwoven web" as used herein means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yarn). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The terms "joined" or "bonded" or "attached", as used herein, encompasses configurations whereby an element is directly secured to another element by affixing the element directly to the other element, and configurations whereby an element is indirectly secured to another element by affixing the element to intermediate member(s) which in turn are affixed to the other element. The terms further include embodiments in which a pocket or other connector is formed in or attached to an area of the absorbent article. Further, these terms include configurations in which the elements are removably, or non-removably, joined, bonded, or attached. For example, wherein an element is described as "joined" within the configuration, it may be either removably joined or non-removably joined unless otherwise specified or evident from the context.

The terms "comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

By "absorbent material" it is meant a material which has some absorbency property or liquid retaining properties, such as SAP, cellulosic fibers as well as synthetic fibers, most preferably is selected from the group consisting of SAP, cellulose (or cellulosic) fibers, and mixtures thereof. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material.

As used herein, the term "absorbent core" refers to the component or components of the article having the most absorbent capacity and comprising an absorbent material and optionally a core wrap enclosing the absorbent material. The term "absorbent core" does not include the acquisition-distribution system or layer or any other component of the article which is not either integral part of the core wrap or placed within the core wrap. The core may consist essentially of, or consist of, a core wrap, absorbent material as defined below and glue enclosed within the core wrap.

The values indicated herein are measured according to the methods indicated herein below, unless specified otherwise. All measurements are performed at $21\pm2°$ C. and $50\pm20\%$ RH, unless specified otherwise. All samples should be kept at least 24 hours in these conditions to equilibrate before conducting the tests, unless indicated otherwise. All measurements should be reproduced on at least 4 samples and the average value obtained indicated, unless otherwise indicated.

THE ABSORBENT ARTICLE

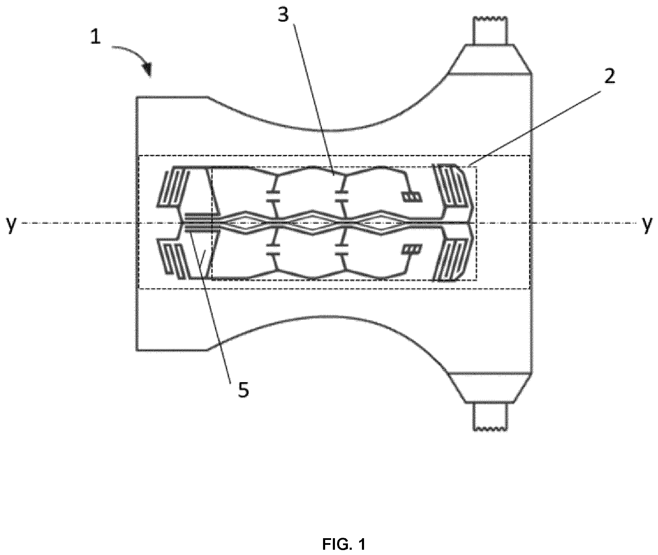

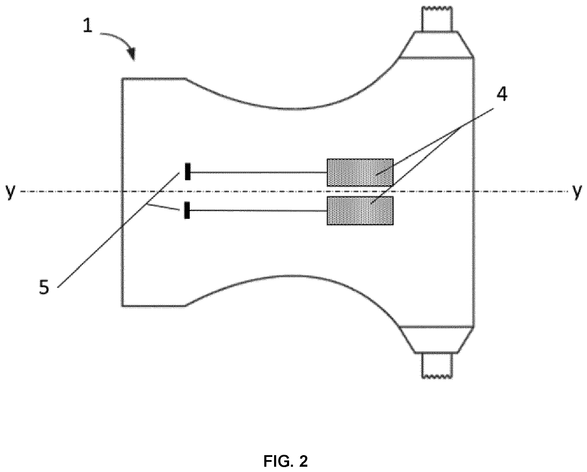

As exemplified in Fig.1 to Fig.3, absorbent articles herein are for personal hygiene such as a diaper, or pant, or incontinence insert or pad or liner, the absorbent article comprising: a topsheet; a backsheet, wherein the topsheet is liquid permeable and the backsheet is substantially liquid impermeable; an absorbent core (2) positioned between said topsheet and backsheet; a first indicator (3) for indicating the presence of exudates and being positioned on a body-facing side of said backsheet, said first indicator (3) comprising an electrically conductive material, wherein said article (1) comprises a second indicator (4) for indicating the presence of exudates and being positioned on a garment-facing side of said backsheet such that the first and second indicators (3, 4) are separated from each other at least in a thickness direction (i.e. a direction substantially following a z-axis that crosses the topsheet, absorbent core, and backsheet; and that may be substantially perpendicular to the longitudinal axis), said second indicator (4) comprising an electrically conductive material, and wherein said first indicator (3) comprises a first application pattern and said second indicator (4) comprises a second application pattern, wherein said first and second application patterns are different. Advantageously, this arrangement allows for accurate automated urine detection by the first indicator and stool detection by the second indicator whilst minimising interference effects in readings which may otherwise result in undesirable false positives being triggered.

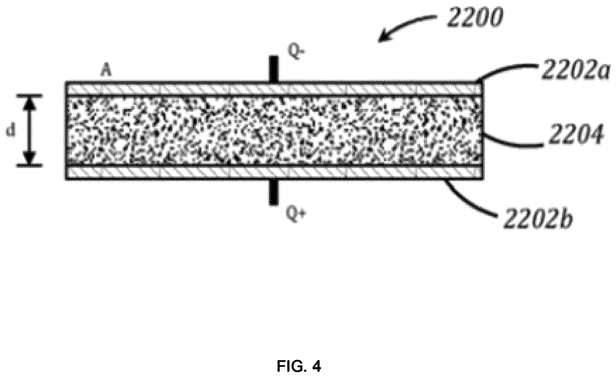

Fig.4 shows a cross-sectional view of an exemplary capacitor 2200. Capacitor 2200 includes two metal conductive plates 2202a and 2202b, which are separated by a dielectric material 2204.

The capacitance C is given by:

$$C = \frac{\varepsilon_0 \varepsilon_r A}{d},$$

where:

A is the area of overlap between the two plates;

$\varepsilon_r$ is the relative static permittivity (also known as the dielectric constant) of the material between the plates (for a vacuum, $\varepsilon_r = 1$);

$\varepsilon_0$ is the electric constant ($\varepsilon_0 \approx 8.854 \times 10^{-12}$ F m-1); and

d is the distance between the plates.

Urine and stool may be considered as having the characteristics of an electrolyte solution. Hence, they may influence the electrical field in their surroundings. The second indicator (4) and the secretion/exudate (e.g. stool) serve here as the 2202b and 2202a plates of capacitor 2200 of Fig.4 accordingly. The backsheet (and/or outer cover) with internal diaper absorption material such as the absorbent core serves here as dielectric layer 2204 of capacitor 2200 of Fig.4. Such configuration implements the physics of a capacitor and may be referred to as a diaper capacitor. Thus, the diaper capacitor may be connected in series with capacitive element of detector.

In order to detect the capacitance in steady state and during change, the detector may be coupled with suitable electronics. The electronics are either embedded in the diaper during the manufacturing process, or externally coupled with the diaper, e.g. with the outer shell of the diaper and/or applied thereto by other means such as printing and the like.

Since secretion appears to be a part of the capacitor of the diaper, it may immediately influence the capacitance magnitude of capacitive detector.

In an embodiment, a photodetector is used as a sensor for detecting urine secretion and/or stool secretion. As urine and/or stool are secreted into the diaper, the amount of light reaching a photodetector decreases, since a greater portion of the light may now be blocked by the secretions. Generally, the amount of light reaching the photodetector changes as a function of the amount of urine and/or stool disposed inside the diaper. An LDR sensor may be based on the principle of a decreasing resistance when light incidence increases.

A circuit may comprise an LDR detector and/or a capacitor connected to operational amplifier converter/conditioner. The operational amplifier may have high input impedance and unity gain, and if LDR is used, the principle may be based on a voltage divider between a fixed resistor, referred also as $R_m$, and LDR, referred also as $R_{photo}$. The output voltage $V_{out}$ may be given by:

$$V_{out} = \frac{V_{CC}}{(1 + R_m/R_{photo})},$$

i.e. output voltage is rather linear to LDR resistance. Alternatively, an integrator circuit including capacitive sensor, resistor and amplifier may be used (instead of resistor and LDR). The value of the output voltage (indicated $V_{out}$) depends on the value of capacitive sensor charge or discharge as a function of time.

An LDR and a 2MΩ resistor may serve as a voltage divider. When light level is low (in our case, when pieces are interlocked), the resistance of LDR may be high. This may prevent current from flowing to the base of the transistor. Consequently, the output voltage may be low, commonly close to 0 volts. However, when light illuminates the LDR without much interference (e.g. in case, when pieces are not interlocked) the resistance may fall and current may flow into the base of transistor, increasing the output voltage to high level (e.g. about 5 volts). Alternatively, an integrator circuit including capacitive sensor, resistor and amplifier may be used (instead of resistor and LDR).

The absorbent article may comprises one or more indicator(s) adapted to indicate the presence and/or absence of bodily exudates and/or determine saturation level and when the article should be replaced with a new clean one. The indicator, in some embodiments, for example, may comprise an indicator that reacts to the presence and/or absence of bodily exudate(s) and/or one or more properties of those bodily exudate(s) within the absorbent article via one or more change in property of the indicator (e.g., a physical, chemical or biological property such as color, smell, sound, pH, or the like). One or more property or state of the indicator, in turn, may be detected by a detector device physically and/or communicatively coupled to the absorbent article. In one particular implementation, for example, the indicator comprises an optical property changing composition or device (e.g., a color-changing composition or device, such as a color changing indicator) that changes an optical property (e.g., color) in response to a variation of pH associated with the presence and/or absence of bodily exudates within the absorbent article). The indicator might also comprise one or more additional indicators of the same or different type that provide different types of indications and/or indications of bodily exudates (or properties of bodily exudates) detected in one or more different regions of the absorbent article. In one embodiment, for example, a second electrical indicator may comprise a resistance, capacitance, inductance or continuity sensitive indicator. Alternatively, such electrical indicators may be provided as an alternative or in conjunction with optical indicators. A resistance sensitive indicator can be provided, for example, by providing two or more electrical conductors disposed at a given spatial distance relative to each other. If bodily exudates, which typically comprise a liquid portion, come in contact with the two electrical conductors, the resistance between the two electrical conductors is reduced (examples of this arrangement are described in more detail in applicant's prior disclosures such as EP3415130, WO/2018/228822, WO/2018/229017, EP3461257, and EP3451988). Other indicators, as known in the field in the context for sensor for absorbent articles, can also be useful. In one particular embodiment, for example, the multiple property changing indicators may be provided in the same or different locations within the absorbent article. For example, an optical property changing indicator (e.g., color changing indicator) may be disposed in a first location of an absorbent article and a second property changing indicator that is the same or a different type of indicator (e.g., another optical property changing indicator such as a color changing indicator) may be disposed in a second location of the absorbent article.

The absorbent article and the one or more indicators may be provided to form an integral unit. For forming the integral unit, the indicator(s) can be directly or indirectly attached to the absorbent article. Direct or indirect attachment to the article is typically to one or more distinguishable element of the article. For example, it can be useful to attach the indicator(s) to the back sheet of the article, such that the indicator(s) and the back sheet of the article from one integral unit. For example if the indicator(s) are provided in sheet form, the respective sheet can be adhesively attached to the back sheet of the article. The respective sheet could also be provided from one and the same material with the back sheet, this material however being treated in suitable ways as to provide an indicator in a pre-defined area (for example by printing an electrically conductive material on a skin or body facing side of the liquid impermeable backsheet layer).

According to one particular embodiment, a detector device (herein also referred to a clip-on processing unit or clip-on unit) is also provided. The detector device, in this implementation, comprises a housing and is adapted to be physically coupled to the absorbent article such that the detector device is further communicatively coupled to one or more indicator integral with the absorbent article. The detector device and/or the absorbent article may comprise one or more connector for removably joining the detector device with the absorbent article. The connector(s) are provided such that the detector device can be attached to the absorbent article and can be detached from the absorbent article including the one or more indicator(s). The detector device can be attached to the integral unit and can be detached from the integral unit. In one particular embodiment, for example, the detector device can be attached to an area of the absorbent article juxtaposed the indicator integral to the absorbent article, and can be detached from that area of the absorbent article so as to be removably connectable thereto.

The housing of the detector device, in one embodiment, has an outer extension in a first direction and an outer extension in a second direction, which is perpendicular to the first direction. The first direction, in this embodiment, may be chosen as characteristic directions, e.g. along a main axis and normally as that of largest extension of the housing. For safety and convenient handling of the device, it may be useful that the device has a length in the first direction of at least 3 cm, 4 cm or more (but normally less than 10 cm) and that the device has a length in the second direction of at least 2 cm, 3 cm or more (but normally less than 10 cm). In one particular embodiment, for example, the housing has a first dimension of at least about 4cm and a second dimension of at least about 8cm. In various embodiments, the housing can be rigid or at least partially or fully flexible. To be flexible the detector device can incorporate flexible electronic components (and boards).

According to one embodiment, the detector device comprises one or more optical sensor, such as a color sensor. This optical sensor can generate an output which depends on an optical property (e.g., a color) observed by the optical sensor. Some examples of optical sensors across a range of wavelengths are: electron tube detectors, photosensors, photomultiplier tubes, phototubes, photodetectors, opto-semiconductor detectors, photodiodes, photomultipliers, image sensors, infrared detectors, thermal sensors, illuminance sensors, visible light sensors and color sensors. In one particular embodiment, for example, the optical sensor may comprise a photodiode such as a TCS 34725 color sensor commercially available from AMS-TAOS USA Inc.

In other embodiments, for example, the detector device need not include a light source, such as where sufficient ambient light may be provided in an application, where light is provided elsewhere (e.g., associated with an absorbent article or clothing, or elsewhere in an environment) or where the property change of a property changing indicator may be detectable without light, such as resistance, impedance and/or capacitance measurements when the indicator comprises an electrically conductive material (such as a conductive ink).

Often, but not necessarily, the detector device will also comprise one or more light, such as a light emitting diode (LED), organic light emitting diode (OLED), an incandescent light bulb, thermionic light emission, luminescence (e.g., among others, fluorescence, chemiluminescence, electroluminescence (e.g., LED), for emitting light onto an area, the wavelength or spectrum of which is to be assessed by the optical sensor. The optical sensor in some color detecting embodiments can be optimized for assessing a color of a color-changing indicator. The optical sensor can be sensitive to visible and non-visible light, namely light in the near IR range. In various embodiments, UV, visible infrared and near infrared wavelengths may be used. A color changing indicator can change its color, for example, based on the presence and/or absence of bodily exudates and/or in response to some other condition being monitored with respect to the absorbent article. In this embodiment, the color sensor can provide an output that varies depending on the presence or absence of bodily exudates.

In a preferred embodiment, the absorbent article further comprises a detection device that can be removably attached to the absorbent article such to become in electrical communication with the first and/or second indicators (3, 4) when attached to said article to measure a change in resistance and/or capacitance in response to a voiding event. A single such detection device connecting to all indicators or one detection device for each of the indicators may be used. Suitable detection devices for use herein are for example described in EP3906907 A1.

Preferably, the first application pattern is applied over a first area of the backsheet and the second application pattern is applied over a second area of the backsheet, and wherein the first area is greater than the second area, preferably wherein the first area extends up to at least proximal to and/or adjacent one or more perimeter edges of the absorbent core (2), and typically even extends beyond said perimeter edges, and the second area is inboard and distal from one or more perimeter edges of the absorbent core (2) such that it is proximal to and/or adjacent and/or overlaps with at least a portion of the longitudinal axis (y) extending substantially parallel to a longest dimension of said article (1). The second area may fall within and/or be positioned inboard of the first area. Preferably wherein the second area is located, typically only, at a back portion of the absorbent article. The back portion typically being delimited by a transversal centreline of the absorbent article (being perpendicular to the longitudinal axis y) and a back transversal edge of the article opposite and substantially parallel thereto.

In an embodiment, the pattern of the first indicator (3) comprises a plurality of sensing tracks, preferably wherein each track forms an open and/or closed circuit corresponding to a different position of the backsheet and the absorbent core such that voiding events in said different positions may be individually monitored; and wherein the pattern of the second indicator (4) comprises one or more sensing tracks having a shape selected from the group consisting of linear, polygonal, and spiral-shaped. Preferably, each of the sensing tracks comprises a connection end (5) arranged to couple to a respective terminal end of the detection device to form an electrical connection between said detection device and said sensing tracks of the indicator. Advantageously this allows for wide coverage for the urine detection which may also permit more accurate warnings to be triggered in case of higher risk of leakage, and targeted detection of stool at about the insult position.

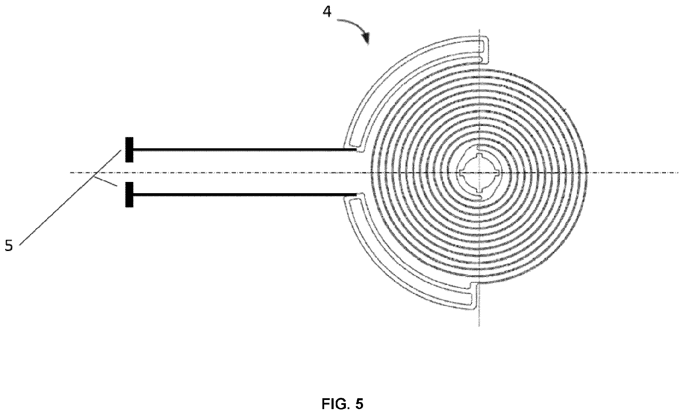

Fig. 5 exemplifies a spiral-shaped second indicator (4) that may be used in absorbent articles herein. Advantageously this arrangement allows to produce a sufficiently strong electric field for capacitance measurements whilst covering a larger surface area under an insult position yet using a lower total amount of conductive material.

In an embodiment, the first and/or second indicators (3, 4) are printed with a conductive ink, preferably wherein the conductive ink is a carbon-based ink and/or a conductive polymer-based ink. Preferably, the carbon-based ink comprises a conductive compound selected from the group consisting of graphene, graphite, nano-carbon-tubes and mixtures thereof. Preferably, the conductive polymer-based ink comprises a polymer selected from the group consisting of polyacetylene, polypyrrole, polyaniline and copolymers thereof, more preferably selected from the group consisting of poly(pyrrole)s (PPY), polyanilines (PANI), poly(thiophene)s (PT), poly(p-phenylene sulfide) (PPS), poly(p-phenylene) (PPP), Poly(acetylene)s (PAC), Poly(p-phenylene vinylene) (PPV), poly(3,4-ethylenedioxythiophene) (PEDOT), and mixtures thereof, most preferred conductive polymer-based ink comprising poly(3,4-ethylenedioxythiophene) polystyrene sulfonate (PEDOT: PSS).

In an embodiment, the indicators (3, 4) are free of metals.

In an embodiment, the absorbent article further comprises a color-changing wetness indicator. When such wetness indicator is present, the detection device may further comprise an optical sensor and one or more light sources that may be as described herein.

In an embodiment, the first indictor (3) is an electrically conductive sensor adapted to change one or more of its electrical properties in response to a voiding event such as presence of exudates, preferably urine, in the absorbent article. Preferably, the one or more electrical properties is resistance; or resistance combined with one or more of capacitance, inductance, and combinations thereof. Preferably, the first indicator (3) is adapted to detect each voiding event of a plurality of, typically sequential, voiding events throughout a wearing period of said absorbent article by a wearer.

In an embodiment, the second indicator (4) is an electrically conductive sensor adapted to change one or more of its electrical properties in response to a voiding event such as presence of exudates, preferably stool, in the absorbent article, most preferably wherein the one or more electrical properties is capacitance. Advantageously this works synergistically with the at least resistance measurement of the first indicator to help exclude false positives. For example a capacitance trigger by the second indicator combined with an absence of a resistive trigger by the first indicator is more likely to be a stool voiding event that should trigger a higher priority warning for a given patient to be changed.

In an embdoment, the backsheet is breathable and a dielectric coating is comprised on the body-facing or garment-facing side of the backsheet at positions corresponding to, preferably only, the second indicator (4) and/or the second application pattern such that one or more areas neighbouring said second indicator (4) and/or second application pattern are free of said coating. The dielectric coating may be in the form of a varnish or other resin-comprising coating. Advantageously this allows to insulate the surface over which the capacitance measurements are to be made for improved accuracy whilst limiting reduction in breathability of the surface which remains important for skin-care.

Preferably, the first indicator (3) and second indicator (4), and/or the first application pattern and the second application pattern, substantially do not overlap when viewed in a planar direction (e.g. from top or bottom views as shown in figures 1-3). Advantageously this limits electric field interference that may impact accuracy.

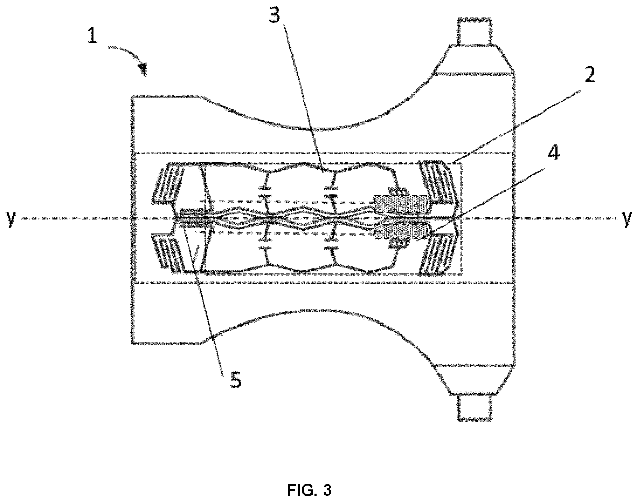

In a preferred embodiment, the first indicator (3) and second indicator (4), and/or the first application pattern and the second application pattern are substantially complementary generally such that when the first indicator (3) and second indicator (4), and/or the first application pattern and the second application pattern are overlayed they substantially fit one within the other. This may be as exemplified in Fig. 3. Advantageously this arrangement limits electric field interference that may impact accuracy as well as optimise surface coverage for exudate detection.

These and other components of the articles will now be discussed in more details.

The topsheet is generally the part of the absorbent article that is directly in contact with the wearer's skin. The topsheet can be joined to the backsheet, the core 2 and/or any other layers as is known in the art. Usually, the topsheet and the backsheet are joined directly to each other in some locations (e.g. on or close to the periphery of the article) and are indirectly joined together in other locations by directly joining them to one or more other elements of the diaper.

The topsheet is preferably compliant, soft-feeling, and non-irritating to the wearer's skin. Further, at least a portion of the topsheet is liquid permeable, permitting liquids to readily penetrate through its thickness. A suitable topsheet may be manufactured from a wide range of materials, such as porous foams, reticulated foams, apertured plastic films, or woven or nonwoven materials of natural fibers (e.g., wood or cotton fibers), synthetic fibers or filaments (e.g., polyester or polypropylene or bicomponent PE/PP fibers or mixtures thereof), or combinations thereof, e.g. a combination of natural and synthetic fibers. A combination of materials can be achieved by combining at least two materials by means of needle punching, ultra-sonic bonding, ring rolling, embossing, gluing or other types of mechanical entanglement. The resulting material may maintain a dual/multiple layer structure, but may also loose a structure of distinguishable layers after such process steps. It can also be useful to provide a formed film patch underneath the topsheet.

If the topsheet includes fibers, the fibers may be spunbond, carded, wet-laid, meltblown, hydroentangled, or otherwise processed as is known in the art, in particular spunbond PP nonwoven. A suitable topsheet comprising a web of staple-length polypropylene fibers is manufactured by Veratec, Inc., a Division of International Paper Company, of Walpole, Mass. under the designation P-8.

The topsheet may comprise one or more apertures to ease penetration of exudates therethrough, such as urine and/or feces (solid, semi-solid, or liquid). The size of at least the primary aperture is important in achieving the desired waste encapsulation performance. If the primary aperture is too small, the waste may not pass through the aperture, either due to poor alignment of the waste source and the aperture location or due to fecal masses having a diameter greater than the aperture. If the aperture is too large, the area of skin that may be contaminated by "rewet" from the article is increased. Typically, the total area of the apertures at the surface of a diaper may have an area of between about 10 mm2 and about 50 mm2, in particular between about 15 mm2 and 35 mm2. Examples of apertured topsheet are disclosed in U.S. Pat. No. 6,632,504, assigned to BBANONWOVENS SIMPSONVILLE. WO2011/163582 also discloses suitable colored topsheet having a basis weight of from 12 to 18 gsm and comprising a plurality of bonded points. Each of the bonded points has a surface area of from 2 mm2 to 5 mm2 and the cumulated surface area of the plurality of bonded points is from 10 to 25% of the total surface area of the topsheet. Typical diaper topsheets have a basis weight of from about 10 to about 21 gsm, in particular between from about 12 to about 18 gsm but other basis weights are possible.

The backsheet is generally that portion of the diaper positioned adjacent the garment-facing surface of the absorbent core 2 and which prevents the exudates absorbed and contained therein from soiling articles such as bedsheets and undergarments. The backsheet is typically impermeable to liquids (e.g. urine). The backsheet may for example be or comprise a thin plastic film such as a thermoplastic film having a thickness of about 0.012 mm to about 0.051 mm. Exemplary backsheet films include those manufactured by Tredegar Corporation, based in Richmond, Va., and sold under the trade name CPC2 film. Other suitable backsheet materials may include breathable materials which permit vapors to escape from the diaper 10 while still preventing exudates from passing through the backsheet 24. Exemplary breathable materials may include materials such as woven webs, nonwoven webs, composite materials such as film-coated nonwoven webs, microporous films such as manufactured by Mitsui Toatsu Co., of Japan under the designation ESPOIR NO and by Tredegar Corporation of Richmond, Va., and sold under the designation EXAIRE, and monolithic films such as manufactured by Clopay Corporation, Cincinnati, Ohio under the name HYTREL blend P18-3097. Some breathable composite materials are described in greater detail in PCT Application No. WO 95/16746 published on Jun. 22, 1995 in the name of E.I. DuPont; U.S. Pat. No. 5,938,648 to LaVon et al., U.S. Pat. No. 4,681,793 to Linman et al., U.S. Pat. No. 5,865,823 to Curro; and U.S. Pat. No. 5,571,096 to Dobrin et al, U.S. Pat. No. 6,946,585B2 to London Brown.

In a preferred embodiment the backsheet is breathable. Breathable backsheet herein typically means that it comprises micro-openings sized to allow at least some water vapour to permeate through the backsheet that typically comprises a film such as a polyethylene (PE) film.

Preferably a backsheet is breathable when the water vapour transmission rate (WVTR) of the backsheet is at least 500 grams/m2 - 24 hours, preferably at least 1,000 grams/m2 - 24 hours, even more preferably from 1,200 grams/m2 - 24 hours to 2,500 grams/m2 - 24 hours, even more preferably from 1,500 grams/m2 - 24 hours to 2,100 grams/m2 - 24 hours, as measured according to ASTM D6701-21.

The backsheet may be joined to the topsheet, the absorbent core 2 or any other element of the diaper by any attachment means known in the art. Suitable attachment means are described above with respect to means for joining the topsheet to other elements of the diaper. For example, the attachment means may include a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minn. and marketed as HL-1620 and HL 1358-XZP. Alternatively, the attachment means may comprise heat bonds, pressure bonds, ultrasonic bonds, dynamic mechanical bonds, or any other suitable attachment means or combinations of these attachment means as are known in the art.

The absorbent core 2 of the absorbent article may comprise one or more core layers. As explained, the absorbent article may comprise an acquisition distribution system, which will typically consist of one or more layers. Most typically, the layers are arranged above the core layer. Hence, a number of layers can be arranged between the topsheet and the backsheet. The skilled person will usually have no difficulty in distinguishing between these layers. In case of doubt, a core layer can be identified as being a layer which is generally less permeable than a layer forming part of the acquisition-/distribution-system.

Permeability generally refers to the quality of a porous material that causes it to a lower liquid or gases to pass through it. Hence, the layers of the acquisition distribution system should generally be more permeable than the layers of the core system as these layers are meant to distribute liquid to the absorbent core, where the liquid is ultimately stored.

The absorbent core can comprise absorbent material with a varying amount of superabsorbent polymers (herein abbreviated as "SAP"), often enclosed within a core wrap. The SAP content can represent from 0% to 80% by weight of the absorbent material contained in the core wrap. Often an SAP content of 20% to 80% by weight of the absorbent material contained in the core wrap is useful. The core wrap is not considered as absorbent material for the purpose of assessing the percentage of SAP in the absorbent core.

The SAP content may be higher than 30%, for example at least 40%, at least 50%, at least 80% of the weight of the absorbent material contained within the core wrap. The absorbent material may in particular embodiments comprise from 10 to 70, for example 30 to 60 weight percent of natural or synthetic fibers.

The absorbent core may comprise a generally planar top edge and a generally planar bottom edge. In some embodiments, the absorbent material will be advantageously distributed in higher amount towards the front edge than towards the rear edge as more absorbency is required at the front. In other embodiments, typically embodiments for other uses of an absorbent article, such as care of elderly incontinent people versus care of babies, the absorbent material will be advantageously distributed in higher amount towards the rear edge than towards the front edge as more absorbency is required at the rear area.

The core wrap may be formed by two separate sheets of nonwoven material which may be at least partially sealed along the edges of the absorbent core. The core wrap may be at least partially sealed along its front edge, back edge and two longitudinal edges so that substantially no absorbent material leaks out of the absorbent core wrap.

The absorbent core of the absorbent article may further comprise adhesive for example to help immobilizing the SAP within the core wrap and/or to ensure integrity of the core wrap, in particular when the core wrap is made of two or more substrates. Such an adhesive can be provided in the form of fibrous thermoplastic adhesive material.

Adhesive or mechanical bonding (such as ultrasonic bonding, thermal bonding, pressure bonding etc.) may be used for bonding top and bottom core wrap layers to form channels substantially free of absorbent material as described herein.

The fibrous thermoplastic adhesive material may be at least partially in contact with the superabsorbent material in the land areas and at least partially in contact with the substrate layer in the junction areas. This imparts an essentially three-dimensional structure to the fibrous layer of thermoplastic adhesive material, which in itself is essentially a two-dimensional structure of relatively small thickness, as compared to the dimension in length and width directions. Thereby, the fibrous thermoplastic adhesive material may provide cavities to cover the absorbent material in the land area, and thereby immobilizes this absorbent material.

The thermoplastic adhesive material may comprise, in its entirety, a single thermoplastic polymer or a blend of thermoplastic polymers, having a softening point, as determined by the ASTM Method D-36-95 "Ring and Ball", in the range between 50° C. and 300° C., and/or the thermoplastic adhesive material may be a hotmelt adhesive comprising at least one thermoplastic polymer in combination with other thermoplastic diluents such as tackifying resins, plasticizers and additives such as antioxidants.

Superabsorbent material, herein also referred to as superabsorbent polymer material, superabsorbent polymers or SAP, refers to absorbent materials which are cross-linked polymeric materials that can absorb at least 10 times their weight of an aqueous 0.9% saline solution as measured using the Centrifuge Retention Capacity (CRC) test (EDANA method WSP 241.2-05E). The SAP used may in particular have a CRC value of more than 20 g/g, or more than 24 g/g, or of from 20 to 50 g/g, or from 20 to 40 g/g, or 24 to 30 g/g. The SAP useful in example absorbent articles may include a variety of water-insoluble, but water-swellable polymers capable of absorbing large quantities of fluids.

The superabsorbent polymer can be in particulate form so as to be flowable in the dry state. Typical particulate absorbent polymer materials are made of poly(meth)acrylic acid polymers. However, e.g. starch-based particulate absorbent polymer material may also be used, as well polyacrylamide copolymer, ethylene maleic anhydride copolymer, cross-linked carboxymethylcellulose, polyvinyl alcohol copolymers, cross-linked polyethylene oxide, and starch grafted copolymer of polyacrylonitrile. The superabsorbent polymer may be polyacrylates and polyacrylic acid polymers that are internally and/or surface cross-linked.

The SAP useful for example embodiments of absorbent articles may be of numerous shapes. The term "particles" refers to granules, fibers, flakes, spheres, powders, platelets and other shapes and forms known to persons skilled in the art of superabsorbent polymer particles. In some embodiments, the SAP particles can be in the shape of fibers, i.e. elongated, acicular superabsorbent polymer particles. In those embodiments, the superabsorbent polymer particles fibers have a minor dimension (i.e. diameter of the fiber) of less than about 1 mm, usually less than about 500 $\mu$m, and preferably less than 250 $\mu$m down to 50 $\mu$m. The length of the fibers is preferably about 3 mm to about 100 mm. The fibers can also be in the form of a long filament that can be woven.

Typically, SAP are spherical-like particles. In contrast to fibers, "spherical-like particles" have a longest and a smallest dimension with a particulate ratio of longest to smallest particle dimension in the range of 1-5, where a value of 1 would equate a perfectly spherical particle and 5 would allow for some deviation from such a spherical particle. The superabsorbent polymer particles may have a particle size of less than 850 μm, or from 50 to 850 μm, preferably from 100 to 710 μm, more preferably from 150 to 650 μm, as measured according to EDANA method WSP 220.2-05. SAP having a relatively low particle size help to increase the surface area of the absorbent material which is in contact with liquid exudates and therefore support fast absorption of liquid exudates.

The SAP may have a particle sizes in the range from 45 μm to 4000 μm, more specifically a particle size distribution within the range of from 45 μm to about 2000 μm, or from about 100 μm to about 1000, 850 or 600 μm. The particle size distribution of a material in particulate form can be determined as it is known in the art, for example by means of dry sieve analysis (EDANA 420.02 "Particle Size distribution).

The total amount of SAP present in the absorbent core may also vary according to expected usage. Diapers for newborns may require less SAP than infant or adult incontinence diapers. The amount of SAP in the core may be for example comprised from about 2 to 60 g, in particular from 5 to 50 g or 10 to 40 g. The average SAP basis weight within the (or "at least one", if several are present) deposition area of the SAP may be for example of at least 50, 100, 200, 300, 400, 500 or more g/m2. The areas of the channels present in the absorbent material deposition area, if any, are deduced from the absorbent material deposition area to calculate this average basis weight.

The optional core wrap may be made of a single substrate folded around the absorbent material, or may advantageously comprise two (or more) substrates which are attached to another. Typical attachments are the so-called C-wrap and/or sandwich wrap. In a C-wrap, the longitudinal and/or transversal edges of one of the substrate are folded over the other substrate to form flaps. These flaps are then bonded to the external surface of the other substrate, typically by gluing.

The absorbent core may comprise one or more channels substantially free of absorbent material and that may be substantially circumscribed by absorbent material such that they do not extend to one or more of the perimeter edges of said core. Top and bottom core wrap layers may be joined together in one or more attachment zones to form said channels. Advantageously such channels aid to improve liquid distribution over the core.

In a preferred embodiment, top and bottom layers of a core wrap are joined together to form a pocket substantially free of absorbent material. Similar to channels described above, the pocket is preferably (but not necessarily) substantially circumscribed by absorbent material such that they do not extend to one or more of the perimeter edges of said core. The pocket may have a geometry with an aspect ratio approaching 1 (such as from 0.5 to less than 2). Advantageously such pocket allows the stool to be collected and with the weight thereof be pushed closer to the backsheet compared to a core in absence of such pocket (generally in view of the reduced thickness in that region by the fact that substantially no absorbent material is present).

In a preferred embodiment, the pocket is positioned such that it substantially superposes the second indicator (4). Advantageously this allows to reduce the power source required to generated the electric field desired for stool detection by e.g. capacitative measurements.

If the core wrap comprises a first substrate and a second substrate these may be made of the same type of material, or may be made of different materials or one of the substrate may be treated differently than the other to provide it with different properties. As the polymers used for nonwoven production are inherently hydrophobic, they are preferably coated with hydrophilic coatings if placed on the fluid receiving side of the absorbent core. It is advantageous that the top side of the core wrap, i.e. the side placed closer to the wearer in the absorbent article, be more hydrophilic than the bottom side of the core wrap. A possible way to produce nonwovens with durably hydrophilic coatings is via applying a hydrophilic monomer and a radical polymerization initiator onto the nonwoven, and conducting a polymerization activated via UV light resulting in monomer chemically bound to the surface of the nonwoven. An alternative possible way to produce nonwovens with durably hydrophilic coatings is to coat the nonwoven with hydrophilic nanoparticles.

The absorbent article may comprise a pair of barrier leg cuffs. The barrier leg cuffs can be formed from a piece of material, typically a nonwoven, which is partially bonded to the rest of the article so that a portion of the material, the barrier leg cuffs, can be partially raised away and stand up from the plane defined by the topsheet when the article is pulled flat as. The barrier leg cuffs can provide improved containment of liquids and other bodily exudates approximately at the junction of the torso and legs of the wearer.

The barrier leg cuffs can be integral with the topsheet or the backsheet, or more typically be formed from a separate material joined to the rest of the article. Typically the material of the barrier leg cuffs may extend through the whole length of the diapers but is "tack bonded" to the topsheet towards the front edge and back edge of the article so that in these sections the barrier leg cuff material remains flush with the topsheet. Each barrier leg cuff may comprise one, two or more elastic close to this free distal edge to provide a better seal.

In addition to the barrier leg cuffs, the article may comprise gasketing cuffs, which are joined to the chassis of the absorbent, article, in particular the topsheet and/or the backsheet and are placed transversely outwardly relative to the barrier leg cuffs. The gasketing cuffs can provide a better seal around the thighs of the wearer. Usually each gasketing leg cuff will comprise one or more elastic string or elastic element comprised in the chassis of the diaper for example between the topsheet and backsheet in the area of the leg openings.

Embodiments of the absorbent articles may comprise an acquisition-distribution layer or system (herein "ADL") and may be a single layer (preferably a spunbond or carded thermobonded layer, or an air-layed nonwoven layer) or may be multilayer and comprising one or more of the following nonwovnen layers: spunbond, carded thermobonded, meltblown, and combinations thereof. The function of the ADL is to quickly acquire the fluid and distribute it to the absorbent core in an efficient manner. The ADL may comprise one, two or more layers, which may form a unitary layer or remain discrete layers which may be attached to each other. In the examples below, the ADL can comprise two layers: a distribution layer and an acquisition layer disposed between the absorbent core and the topsheet, but embodiments of absorbent articles are not restricted to this example and rather multilayer or single-layer ADLs are equally contemplated herein.

Typically, the ADL will not comprise SAP as this may slow the acquisition and distribution of the fluid.

The distribution layer and the acquisition layer may comprise the same or different layers. For example the distribution layer may be a nonwoven selected from a spunbond, meltblown, carded (preferably thermobonded), and combinations therof.

The ADL may comprise an acquisition layer. The acquisition layer may be disposed between the distribution layer and topsheet. The acquisition layer may typically be or comprise a non-woven material, for example a SMS or SMMS material, comprising a spunbonded, a melt-blown and a further spunbonded layer or alternatively a carded chemical-bonded nonwoven.

The absorbent article may include a fastening system. The fastening system can be used to provide lateral tensions about the circumference of the absorbent article to hold the absorbent article on the wearer as is typical for taped diapers. This fastening system is not necessary for training pant article since the waist region of these articles is already bonded. The fastening system usually comprises a fastener such as tape tabs (also referred to as adhesive tabs), hook and loop fastening components, interlocking fasteners such as tabs & slots, buckles, buttons, snaps, and/or hermaphroditic fastening components, although any other known fastening means are generally acceptable. A landing zone is normally provided on the front waist region for the fastener to be releasably attached.

The absorbent article may comprise front ears and back ears as is known in the art. The ears can be integral part of the chassis, for example formed from the topsheet and/or backsheet as side panel. Alternatively, they may be separate elements attached by gluing and/or heat embossing or pressure bonding. The back ears are advantageously stretchable to facilitate the attachment of the adhesive tabs on the landing zone and maintain the taped diapers in place around the wearer's waist. The back ears may also be elastic or extensible to provide a more comfortable and contouring fit by initially conformably fitting the absorbent article to the wearer and sustaining this fit throughout the time of wear well past when absorbent article has been loaded with exudates since the elasticized ears allow the sides of the absorbent article to expand and contract.

The absorbent article may also comprise at least one elastic waist feature that helps to provide improved fit and containment. The elastic waist feature is generally intended to elastically expand and contract to dynamically fit the wearer's waist. The elastic waist feature preferably extends at least longitudinally outwardly from at least one waist edge of the absorbent core 2 and generally forms at least a portion of the end edge of the absorbent article. Disposable diapers can be constructed so as to have two elastic waist features, one positioned in the front waist region and one positioned in the back waist region. The elastic waist feature may be constructed in a number of different configurations including those described in U.S. Pat. Nos. 4,515,595, 4,710,189, 5,151,092 and 5,221,274.

Typically, adjacent layers and components will be joined together using conventional bonding method such as adhesive coating via slot coating or spraying on the whole or part of the surface of the layer, or thermobonding, or pressure bonding or combinations thereof.

KIT OF PARTS

In a further aspect the disclosure relates to a kit comprising a plurality of absorbent articles herein described and a detection device operable with any one of said absorbent articles. Preferably wherein the plurality of absorbent articles comprises absorbent articles having at least two, preferably at least three, different absorbancy levels and/or sizes. Advantageously this allows for ease of use and replenishment for stock management purposes.

**Claims**

1. An absorbent article (1) for personal hygiene comprising:

   a topsheet;
   a backsheet, wherein the topsheet is liquid permeable and the backsheet is substantially liquid impermeable;
   an absorbent core (2) positioned between said topsheet and backsheet,
   a first indicator (3) for indicating the presence of exudates and being positioned on a body-facing side of said backsheet, said first indicator (3) comprising an electrically conductive material, **characterized in that** said article (1) comprises a second indicator (4) for indicating the presence of exudates and being positioned on a garment-facing side of said backsheet such that the first and second indicators (3, 4) are separated from each other at least in a thickness direction, said second indicator (4) comprising an electrically conductive material, and **in that** said first indicator (3) comprises a first application pattern and said second indicator (4) comprises a second application pattern, wherein said first and second application patterns are different.

2. An absorbent article (1) according to Claim 1 further comprising a detection device that can be removably attached to the absorbent article such to become in electrical communication with the first and/or second indicators (3, 4) when attached to said article to measure a change in resistance and/or capacitance in response to a voiding event.

3. An absorbent article (1) according to any of the preceding Claims wherein the first application pattern is applied over a first area of the backsheet and the second application pattern is applied over a second area of the backsheet, and wherein the first area is greater than the second area, preferably wherein the first area extends to a position at least proximal to and/or adjacent one or more perimeter edges of the absorbent core (2) and the second area is inboard and distal from one or more perimeter edges of the absorbent core (2) such that it is proximal to and/or adjacent and/or overlaps a longitudinal axis (y) extending substantially parallel to a longest dimension of said article (1).

4. An absorbent article according to any of the preceding Claims, wherein the pattern of the first indicator (3) comprises a plurality of sensing tracks, preferably wherein each track forms an open and/or closed circuit corresponding to a different position of the backsheet and the absorbent core such that voiding events in said different positions may be individually monitored; and wherein the pattern of the second indicator (4) comprises one or more sensing tracks having a shape selected from the group consisting of linear, polygonal, and spiral-shaped.

5. An absorbent article according to Claim 4, wherein each of the sensing tracks comprises a connection end (5) arranged to couple to a respective terminal end of the detection device to form an electrical connection between said detection device and said sensing tracks of the indicator.

6. An absorbent article according to any of the preceding Claims wherein the first and/or second indicators (3, 4) are printed with a conductive ink, preferably wherein the conductive ink is a carbon-based ink and/or a conductive polymer-based ink.

7. An absorbent article according to Claim 6 wherein the carbon-based ink comprises a conductive compound selected from the group consisting of graphene, graphite, nano-carbon-tubes and mixtures thereof; and/or wherein the conductive polymer-based ink comprises a polymer selected from the group consisting of polyacetylene, polypyrrole, polyaniline and copolymers thereof, more preferably selected from the group consisting of poly(pyrrole)s (PPY), polyanilines (PANI), poly(thiophene)s (PT), poly(p-phenylene sulfide) (PPS), poly(p-phenylene) (PPP), Poly(acetylene)s (PAC), Poly(p-phenylene vinylene) (PPV), poly(3,4-ethylenedioxythiophene) (PEDOT), and mixtures thereof, most preferred conductive polymer-based ink comprising poly(3,4-ethylenedioxythiophene) polystyrene sulfonate (PEDOT:PSS).

8. An absorbent material according to any of the preceding Claims, wherein the indicators (3, 4) are free of metals.

9. An absorbent article according to any of the preceding Claims wherein the first indicator (3) is an electrically conductive sensor adapted to change one or more of its electrical properties in response to a voiding event such as presence of exudates, preferably urine, in the absorbent article.

10. An absorbent article according to Claim 9 wherein the one or more electrical properties is resistance; or resistance combined with one or more of capacitance, inductance, and combinations thereof.

11. An absorbent article according to Claims 9 to 10 wherein the first indicator (3) is adapted to detect each voiding event of a plurality of, typically sequential, voiding events throughout a wearing period of said absorbent article by a wearer.

12. An absorbent article according to any of the preceding Claims where in the second indicator (4) is an electrically conductive sensor adapted to change one or more of its electrical properties in response to a voiding event such as presence of exudates, preferably stool, in the absorbent article, most preferably wherein the one or more electrical properties is capacitance.

13. An absorbent article according to any of the preceding Claims wherein the backsheet is breathable and a dielectric coating is comprised on the body-facing or garment-facing side of the backsheet at positions corresponding to, preferably only, the second indicator (4) and/or the second application pattern such that one or more areas neighbouring said second indicator (4) and/or second application pattern are free of said coating.

**14.** An absorbent article according to any of the preceding Claims wherein the first indicator (3) and second indicator (4), and/or the first application pattern and the second application pattern, substantially do not overlap when viewed in a planar direction.

**15.** An absorbent article according to any of the preceding Claims wherein the first indicator (3) and second indicator (4), and/or the first application pattern and the second application pattern, are substantially complementary.

**Patentansprüche**

**1.** Absorbierender Artikel (1) zur persönlichen Hygiene, umfassend:

eine obere Folie;
eine hintere Folie, wobei die obere Folie flüssigkeitsdurchlässig ist und die hintere Folie im Wesentlichen flüssigkeitsundurchlässig ist;
einen absorbierenden Kern (2), der zwischen der oberen Folie und der hinteren Folie positioniert ist,
eine erste Anzeige (3), um die Anwesenheit von Exsudaten anzuzeigen, und positioniert auf einer auf den Körper gerichteten Seite der hinteren Folie, wobei die erste Anzeige (3) eine elektrisch leitfähiges Material umfasst,
**dadurch gekennzeichnet, dass** der Artikel (1) eine zweite Anzeige (4) umfasst, um die Anwesenheit von Exsudaten anzuzeigen, und positioniert auf einer auf die Kleidung gerichteten Seite der hinteren Folie, so dass die erste und zweite Anzeige (3, 4) voneinander mindestens in einer Dickenrichtung getrennt sind, wobei die zweite Anzeige (4) ein elektrisch leitfähiges Material umfasst, und dadurch, dass die erste Anzeige (3) ein erstes Anwendungsmuster umfasst und die zweite Anzeige (4) ein zweites Anwendungsmuster umfasst, wobei das erste und das zweite Anwendungsmuster verschieden sind.

**2.** Absorbierender Artikel (1) nach Anspruch 1, weiter umfassend eine Nachweisvorrichtung, die entfernbar an den absorbierenden Artikel derart befestigt werden kann, dass sie mit der ersten und/oder zweiten Anzeige (3, 4) in elektrische Kommunikation gelangt, wenn sie an dem Artikel befestigt ist, um eine Änderung des Widerstands und/oder der Kapazität in Reaktion auf ein Entleerungsereignis zu messen.

**3.** Absorbierender Artikel (1) nach einem der vorhergehenden Ansprüche, wobei das erste Anwendungsmuster auf einen ersten Bereich der hinteren Folie angewendet wird und das zweite Anwendungsmuster auf einen zweiten Bereich der hinteren Folie angewendet wird, und wobei der erste Bereich grösser als der zweite Bereich ist, vorzugsweise wobei sich der erste Bereich in eine Position erstreckt, die mindestens proximal zu und/oder benachbart einer oder mehrerer Umfangskanten des absorbierenden Kerns (2) ist, und der zweite Bereich innerhalb und distal von einer oder mehreren Umfangskanten des absorbierenden Kerns (2) ist, so dass er proximal zu und/oder benachbart zu einer Längsachse (y) ist und/oder diese überlappt und sich im Wesentlichen parallel zu einer längsten Abmessung des Artikels (1) erstreckt.

**4.** Absorbierender Artikel nach einem der vorhergehenden Ansprüche, wobei das Muster der ersten Anzeige (3) eine Vielzahl von Abtastspuren umfasst, vorzugsweise wobei jede Abtastspur eine offene und/oder geschlossene Schaltung bildet, die einer verschiedenen Position der hinteren Folie und des absorbierenden Kerns entspricht, so dass Entleerungsereignisse in den verschiedenen Positionen individuell überwacht werden können, und wobei das Muster der zweiten Anzeige (4) eine oder mehrere Abtastspuren umfasst, die eine Form aufweisen, ausgewählt aus der Gruppe, bestehend aus linear, polygonal und spiralförmig.

**5.** Absorbierender Artikel nach Anspruch 4, wobei jede der Abtastspuren ein Verbindungsende (5) umfasst, das angeordnet ist, um ein entsprechendes Terminalende der Erkennungsvorrichtung zu koppeln, um eine elektrische Verbindung zwischen der Erkennungsvorrichtung und den Abtastspuren der Anzeige zu bilden.

**6.** Absorbierender Artikel nach einem der vorhergehenden Ansprüche, wobei die erste und/oder die zweite Anzeige (3, 4) mit einer leitfähigen Tinte gedruckt sind, vorzugsweise wobei die leitfähige Tinte eine kohlenstoffbasierte Tinte und/oder eine leitfähige polymerbasierte Tinte ist.

**7.** Absorbierender Artikel nach Anspruch 6, wobei die kohlenstoffbasierte Tine eine leitfähige Verbindung umfasst, ausgewählt aus der Gruppe, bestehend aus Graphen, Graphit, Nanokohlenstoffröhrchen und Mischungen daraus; und/oder wobei die leitfähige polymerbasierte Tinte ein Polymer umfasst, ausgewählt aus der Gruppe, bestehend aus Polyacetylen, Polypyrrol, Polyanilin und Copolymeren davon, insbesondere ausgewählt aus der Gruppe, bestehend aus Polypyrrol(en) (PPY), Polyanilinen (PANI), Poly(thiophen)en (PT), Poly(p-phenylensulfid) (PPS), Poly(p-phenylen) (PPP), Polyacetylen(en) (PAC), Poly(p-phenylenvinylen) (PPV), Poly(3,4-ethylendioxythiophen) (PEDOT) und Mischungen daraus, am meisten bevorzugt eine polymerbasierte Tinte, umfassend Poly(3,4-ethylendioxythiophen) polystyrensulfonat (PEDOT:PSS).

8. Absorbierendes Material nach einem der vorhergehenden Ansprüche, wobei die Anzeigen (3, 4) frei von Metallen sind.

9. Absorbierender Artikel nach einem der vorhergehenden Ansprüche, wobei die erste Anzeige (3) ein elektrisch leitfähiger Sensor ist, der ausgelegt ist, um eine oder mehrere seiner elektrischen Eigenschaften in Reaktion auf ein Entleerungsereignis wie z. B. die Anwesenheit von Exsudaten, vorzugsweise Urin, im absorbierenden Artikel zu ändern.

10. Absorbierender Artikel nach Anspruch 9, wobei die eine oder die mehreren elektrischen Eigenschaften Resistenz; oder Resistenz kombiniert mit einem oder mehreren von Kapazität, Induktanz und Kombinationen daraus sind.

11. Absorbierender Artikel nach Anspruch 9 bis 10, wobei die erste Anzeige (3) ausgelegt ist, um jedes Entleerungsereignis einer Vielzahl von typischerweise sequenziellen Entleerungsereignissen während eines Tragezeitraums des absorbierenden Artikels durch einen Träger zu erkennen.

12. Absorbierender Artikel nach einem der vorhergehenden Ansprüche, wobei in der zweiten Anzeige (4) ein elektrisch leitfähiger Sensor ist, der ausgelegt ist, um eine oder mehrere seiner elektrischen Eigenschaften in Reaktion auf ein Entleerungsereignis wie z. B. die Anwesenheit von Exsudaten, vorzugsweise Stuhl, im absorbierenden Artikel zu ändern, am bevorzugtesten, wobei die eine oder die mehreren elektrischen Eigenschaften Kapazität ist/sind.

13. Absorbierender Artikel nach einem der vorhergehenden Ansprüche, wobei die hintere Folie atmungsaktiv ist und eine dielektrische Beschichtung auf der auf den Körper oder auf die Kleidung gerichteten Seite der hinteren Folie an Positionen vorhanden ist, die der, vorzugsweise nur der zweiten, Anzeige (4) und/oder dem zweiten Anwendungsmuster entspricht, so dass ein oder mehrere Bereiche, die der zweiten Anzeige (4) und/oder dem zweiten Anwendungsmuster benachbart sind, frei von der Beschichtung sind.

14. Absorbierender Artikel nach einem der vorhergehenden Ansprüche, wobei die erste Anzeige (3) und die zweite Anzeige (4) und/oder das erste Anwendungsmuster und das zweite Anwendungsmuster im Wesentlichen nicht überlappen, wenn sie in einer planaren Richtung betrachtet werden.

15. Absorbierender Artikel nach einem der vorhergehenden Ansprüche, wobei die erste Anzeige (3) und die zweite Anzeige (4) und/oder das erste Anwendungsmuster und das zweite Anwendungsmuster im Wesentlichen komplementär sind.

**Revendications**

1. Article absorbant (1) pour l'hygiène corporelle comprenant :

une feuille supérieure ;
une feuille arrière, dans lequel la feuille supérieure est perméable aux liquides et la feuille arrière est sensiblement imperméable aux liquides ;
un noyau absorbant (2) positionné entre lesdites feuille supérieure et feuille arrière,
un premier indicateur (3) pour indiquer la présence d'exsudats et étant positionné sur un côté faisant face au corps de ladite feuille arrière, ledit premier indicateur (3) comprenant un matériau électroconducteur,
**caractérisé en ce que** ledit article (1) comprend un deuxième indicateur (4) pour indiquer la présence d'exsudats et étant positionné sur un côté faisant face au vêtement de ladite feuille arrière de telle sorte que les premier et deuxième indicateurs (3, 4) sont séparés l'un de l'autre au moins dans une direction de l'épaisseur, ledit deuxième indicateur (4) comprenant un matériau électroconducteur, et **en ce que** ledit premier indicateur (3) comprend un premier motif d'application et ledit deuxième indicateur (4) comprend un deuxième motif d'application, dans lequel lesdits premier et deuxième motifs d'application sont différents.

2. Article absorbant (1) selon la revendication 1 comprenant en outre un dispositif de détection qui peut être fixé de manière amovible à l'article absorbant de sorte à être en communication électrique avec les premier et/ou deuxième indicateurs (3, 4) lorsqu'il est fixé audit article pour mesurer un changement de résistance et/ou de capacité en réponse à un événement de miction.

3. Article absorbant (1) selon l'une quelconque des revendications précédentes dans lequel le premier motif d'application est appliqué sur une première zone de la feuille arrière et le deuxième motif d'application est appliqué sur une deuxième zone de la feuille arrière, et dans lequel la première zone est supérieure à la deuxième zone, de préférence dans lequel la première zone s'étend vers une position au moins proximale et/ou adjacente à un ou plusieurs bords périphériques du noyau absorbant (2) et la deuxième zone est vers l'intérieur et distale des un ou plusieurs bords périphériques du noyau absorbant (2) de telle sorte qu'elle est proximale et/ou adjacente à et/ou chevauche un axe longitudinal (y) s'étendant sensiblement parallèlement à une dimension la plus longue dudit article (1).

4. Article absorbant selon l'une quelconque des revendications précédentes, dans lequel le motif du premier indicateur (3) comprend une pluralité de pistes de détection, de préférence dans lequel chaque piste forme un circuit ouvert et/ou fermé correspondant à une position différente de la feuille arrière et du noyau absorbant de telle sorte que des événements de miction dans lesdites différentes positions peuvent être surveillés individuellement ; et dans lequel le motif du deuxième indicateur (4) comprend une ou plusieurs pistes de détection ayant une forme sélectionnée dans le groupe consistant en linéaire, polygonale et en spirale.

5. Article absorbant selon la revendication 4, dans lequel chacune des pistes de détection comprend une extrémité de connexion (5) agencée pour se coupler à une extrémité terminale respective du dispositif de détection pour former une connexion électrique entre ledit dispositif de détection et lesdites pistes de détection de l'indicateur.

6. Article absorbant selon l'une quelconque des revendications précédentes dans lequel les premier et/ou deuxième indicateurs (3, 4) sont imprimés avec une encre conductrice, de préférence dans lequel l'encre conductrice est une encre à base de carbone et/ou une encre à base de polymère conducteur.

7. Article absorbant selon la revendication 6 dans lequel l'encre à base de carbone comprend un composé conducteur sélectionné dans le groupe consistant en graphène, graphite, nanotubes de carbone et mélanges de ceux-ci ; et/ou dans lequel l'encre à base de polymère conducteur comprend un polymère sélectionné dans le groupe consistant en polyacétylène, polypyrrole, polyaniline et copolymères de ceux-ci, plus préférablement sélectionné dans le groupe consistant en poly(pyrrole)s (PPY), polyanilines (PANI), poly(thiophène)s (PT), poly(sulfure de p-phénylène) (PPS), poly(p-phénylène) (PPP), Poly(acétylène)s (PAC), Poly(vinylène de p-phénylène) (PPV), poly(3,4-éthylènedioxythiophène) (PEDOT), et les mélanges de ceux-ci, l'encre à base de polymère conductrice préférée entre toutes comprenant du poly(3,4-éthylènedioxythiophène)-sulfonate de polystyrène (PEDOT:PSS).

8. Matériau absorbant selon l'une quelconque des revendications précédentes, dans lequel les indicateurs (3, 4) sont dépourvus de métaux.

9. Article absorbant selon l'une quelconque des revendications précédentes dans lequel le premier indicateur (3) est un capteur électroconducteur adapté pour changer une ou plusieurs de ses propriétés électriques en réponse à un événement de miction tel qu'une présence d'exsudats, de préférence d'urine, dans l'article absorbant.

10. Article absorbant selon la revendication 9 dans lequel les une ou plusieurs propriétés électriques sont la résistance ; ou la résistance combinée à une ou plusieurs de la capacité, l'inductance et des combinaisons de celles-ci.

11. Article absorbant selon les revendications 9 à 10 dans lequel le premier indicateur (3) est adapté pour détecter chaque événement de miction d'une pluralité d'événements de miction, d'ordinaire séquentiels, tout au long d'une période de port dudit article absorbant par un utilisateur.

12. Article absorbant selon l'une quelconque des revendications précédentes dans lequel dans le deuxième indicateur (4) se trouve un capteur électroconducteur adapté pour changer une ou plusieurs de ses propriétés électriques en réponse à un événement de miction tel qu'une présence d'exsudats, de préférence de selles, dans l'article absorbant, de manière préférée entre toutes dans lequel les une ou plusieurs propriétés électriques sont la capacité.

13. Article absorbant selon l'une quelconque des revendications précédentes dans lequel la feuille arrière est respirante et un revêtement diélectrique est présent sur le côté faisant face au corps ou sur le côté faisant face au vêtement de la feuille arrière dans des positions correspondant, de préférence uniquement, au deuxième indicateur (4) et/ou au deuxième motif d'application de telle sorte qu'une ou plusieurs zones voisines dudit deuxième indicateur (4) et/ou dudit deuxième motif d'application sont dépourvues dudit revêtement.

14. Article absorbant selon l'une quelconque des revendications précédentes dans lequel le premier indicateur (3) et le deuxième indicateur (4), et/ou le premier motif d'application et le deuxième motif d'application, ne se chevauchent sensiblement pas lorsqu'ils sont observés dans une direction plane.

15. Article absorbant selon l'une quelconque des revendications précédentes dans lequel le premier indicateur (3) et le deuxième indicateur (4), et/ou le premier motif d'application et le deuxième motif d'application, sont sensiblement complémentaires.

FIG. 1

FIG. 2

FIG. 3

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5151092 A, Buell
- WO 2008155699 A
- WO 2012052172 A
- EP 2496197 B1
- EP 2739254 B1
- EP 2582341 B1
- EP 3415130 A
- WO 2018228822 A
- WO 2018229017 A
- EP 3461257 A
- EP 3451988 A
- EP 3906907 A1

- US 6632504 B
- WO 2011163582 A
- WO 9516746 A
- US 5938648 A, LaVon
- US 4681793 A, Linman
- US 5865823 A, Curro
- US 5571096 A, Dobrin
- US 6946585 B2, London Brown
- US 4515595 A
- US 4710189 A
- US 5221274 A